# EUROPÄISCHE PATENTSCHRIFT

(11) **EP 0 499 176 B1**
(45) Veröffentlichungstag und Bekanntmachung des Hinweises auf die Patenterteilung: **29.12.1997**
(21) Anmeldenummer: 92102187.9
(22) Anmeldetag: 10.02.1992
(51) Int. Cl.: C12P 21/08, C12N 5/10, C12N 5/24, G01N 33/577

(54) **Monoklonale Antikörper gegen humane Pankreas-Inselzellen**
Monoclonal antibodies against human pancreatic islet cells
Anticorps monoclonaux contre les cellules des îlots pancréatiques humains

(30) Priorität: 14.02.1991 DE 4104498; 07.09.1991 DE 4129849
(43) Veröffentlichungstag der Anmeldung: 19.08.1992
(73) Patentinhaber: BOEHRINGER MANNHEIM GMBH, 68298 Mannheim (DE)
(72) Erfinder: Endl, Josef, Dr., W-8120 Weilheim (DE); Brandt, Michael, Dr., W-8127 Iffeldorf (DE); Jungfer, Herbert, Dr., W-8130 Starnberg (DE); Albert, Winfried, Dr., W-8121 Eberfing (DE); Kientsch-Engel, Rosemarie, Dr., W-8133 Feldafing (DE); Scherbaum, Werner, W-7900 Ulm (DE); Richter, Wiltrud, W-7900 Ulm (DE); Eiermann, Thomas, W-7900 Ulm (DE)

(56) Entgegenhaltungen:
- CLINICAL & EXPERIMENTAL IMMUNOLOGY, Band 82, Nr. 1, Oktober 1990, Oxford (GB); E. HOUSSAINT et al., Seiten 44-51/
- METABOLISM, Band 36, Nr. 4, April 1987; K.S. TAN et al., Seiten 327-334/
- NATURE, Band 300, 18 November 1982, London (GB); G.S. EISENBARTH et al., Seiten 264-267/
- NATURE, Band 347, 13 September 1990, London (GB); S. BAEKKESKOV et al., Seiten
- 151-156/
- EMBO Journal, Vol 10, 1275-1284 (1991)

## Beschreibung

Die Erfindung betrifft humane, monoklonale Antikörper, die mit Inselzellen des Pankreas reagieren, deren Verwendung als Standard bei der Bestimmung von Antikörpern gegen ein Inselzellantigen, sowie ein Verfahren zur Bestimmung von Antikörpern gegen ein Inselzellantigen des Pankreas.

In Seren von Prädiabetikern bzw. frisch diagnostizierten Diabetikern findet man häufig Antikörper, die mit den endokrinen Zellen des Pankreas, den Inselzellen, reagieren. Diese Antikörper werden im allgemeinen als islet cell antibodies (ICA) bezeichnet. Die typischen ICA sind vom IgG-Isotyp (G. Botazzo et al., Lancet 2 (1974) 1279 - 1283). Sie reagieren selektiv mit den endokrinen Zellen des Pankreas, nicht jedoch mit den exokrinen Zellen, den Ausführungsgängen oder dem Bindegewebe. Die ebenfalls nachweisbaren autoreaktiven Antikörper vom IgM-Isotyp zeigen dagegen keine präferenzielle Inselzellfärbung, sondern reagieren in gleicher Weise auch mit anderen Geweben, wie Hypophyse, Schilddrüse, T-Lymphozyten und Nebenniere (E. Garzelli et al., J. Clin. Invest. 77, 1986, 1627 - 1631; S. Srikanta et al., Mol. Biol. Med. 3, 1986, 113 - 127). Sie gehören wahrscheinlich zum Typ der wenig affinen natürlichen Autoantikörper geringer Spezifität, die auch im Blut gesunder Personen vorkommen (Seigneurin et al., Blood 71 (1988), 581). Die Anwesenheit der ICA vom IgG-Isotyp im Serum ist dagegen ein prädiktiver Marker für den sogenannten Insulin-abhängigen Diabetes (gleichbedeutend mit juvenilem Diabetes bzw. TypI-Diabetes). Diese Autoantikörper können bis zu neun Jahren vor Ausbruch des Diabetes bereits im Serum nachgewiesen werden.

Die Herstellung von humanen monoklonalen Antikörpern mit Reaktivität gegen Inselzellen wurde bereits mehrfach versucht. G. Eisenbarth et al. (Nature 300, 1982, 264 - 267) erhielten dabei einen monoklonalen Antikörper, der jedoch vom IgM-Isotyp ist. E. Garzelli et al. (J. Clin. Invest. 77, 1986, 1627 - 1631) erhielten Antikörper vom IgM-Isotyp, die außer mit Pankreasgewebe auch mit der Schilddrüse, peripheren Nerven, der Speiseröhre und T-Lymphozyten reagieren. S. Spitalnik et.al. (International Research Symposium "The Immunology of Diabetes", American Diabetes Association Woods Hole, MA, Oct. 27-30 (1987), Abstr. 113) erhielt ebenfalls einen polyreaktiven Antikörper vom IgM-Isotyp. W. Richter et al. (Horm. Metabol. Res. 21, 1989, 686 - 688) gelang es zwar, die Lymphozyten zu immortalisieren, die IgG gegen Inselzellen produzieren, jedoch nicht, diese B-Lymphozyten auf Einzelzellbasis zu klonieren. E. Houssaint et al. (Clin. exp. Immunol. 82 (1990), 44-51) erhielten einen humanen monoklonalen IgG-Antikörper, der jedoch nur eine positive Reaktion gegen Ratten- und Hamster-Inselzellinien zeigt.

Von S. Srikanta und G. Eisenbarth (Mol. Biol. Med. 3 (1986), 113-127) wurden Antigene von Inselzellen beschrieben, die jedoch nicht spezifisch für Inselzellen sind. Die Antigene oder Epitope dieser Antigene sind auch in anderen endokrinen Geweben wie z.B. der Hypophyse, Schilddrüse, Nebenschilddrüse, Thymus, Nebenniere sowie in Melanozyten nachweisbar.

S. Baekkeskov et.al. (Nature 347 (1990), 151-156) identifizierten ein weiteres, 64 kD großes Inselzellantigen als Glutamat-Decarboxylase (GAD). Dieses Antigen wird außer in den Inselzellen des Pankreas auch in den γ-Aminobuttersäure-sezernierenden Neuronen des ZNS mit hoher Rate exprimiert. Patienten mit einer seltenen neurologischen Erkrankung, dem sogenannten "stiff-man-Syndrom", entwickeln Autoantikörper gegen GAD. Fast alle Patienten mit dem "stiff-man-Syndrom" weisen außerdem auch Auto-Antikörper gegen Inselzellen des Pankreas auf, so daß das "stiff-man-Syndrom" häufig von einem Diabetes mellitus Typ I begleitet ist. Aus diesen Beobachtungen wurde die funktionelle Relevanz der GAD als Autoantigen sowohl beim "stiff-man-Syndrom" als auch beim Diabetes mellitus Typ I abgeleitet.

Es war daher die Aufgabe der Erfindung, humane monoklonale Antikörper vom IgG-Isotyp zur Verfügung zu stellen, die mit einem humanen Inselzellantigen reagieren. Weiterhin war es Aufgabe der Erfindung ein Verfahren zur Bestimmung von Antikörpern gegen ein Inselzellantigen zur Verfügung zu stellen.

Diese Aufgabe wird gelöst durch humane monoklonale Antikörper vom IgG-Isotyp gegen humane Pankreas-Inselzellen, erhältlich durch Immortalisieren von humanen Lymphozyten von Prädiabetikern oder Diabetikern, Behandeln des Kulturüberstandes der immortalisierten Zellen mit einem Konjugat aus Antikörpern gegen humanes Fcγ und einer Markierung, anschließende Behandlung mit humanem Immunglobulin, Inkubieren mit immobilisierten humanen Pankreas-Inselzellen oder immobilisierter GAD, Identifizieren einer immortalisierten humanen Zellkultur, die einen Antikörper gegen Pankreas-Inselzellen produziert, über die Bestimmung der an die immobilisierten Inselzellen oder die immobilisierte GAD gebundenen Markierung, Isolieren einer humanen immortalisierten Zelle,die diesen Antikörper produziert, Vermehrung dieser immortalisierten Zelle und Isolierung des von diesen Zellen produzierten monoklonalen Antikörpers.

Es hat sich überraschenderweise gezeigt, daß die erfindungsgemäß erhältlichen monoklonalen Antikörper spezifisch mit gereinigter GAD reagieren. Nach Inkubation mit verschiedenen Gewebeschnitten reagieren die erfindungsgemäßen Antikörper mit Inselzellen des Pankreas und dem Cerebellum, nicht aber mit anderen Geweben wie z. B. aus Nebenniere, Magen, Darm, Hypophyse, Großhirnrinde, Lunge, Leber oder Schilddrüse.

Es hat sich dabei überraschenderweise gezeigt, daß die Bindung der erfindungsgemäßen Antikörper an Pankreasschnitte durch Vorinkubation mit mindestens einem von drei Diabetikerseren abgeschwächt oder ganz verhindert werden kann. Dies zeigt, daß die erfindungsgemäßen Antikörper nicht nur für den Diabetes des speziellen Spenders, aus dessen Blut sie gewonnen wurden, sondern generell für den Diabetes mellitus Typ I relevant sind.

Die Immortalisierung der humanen Lymphozyten von Prädiabetikern oder Diabetikern erfolgt vorzugsweise durch Transformation mit Epstein-Barr-Virus (EBV). Daneben können auch andere, dem Fachmann geläufige Verfahren (z. B. Hybridomtechnik) verwendet werden.

Zum Nachweis von immortalisierten Zellen, die den gewünschten Antikörper produzieren, wird ein Konjugat aus Antikörpern gegen humanes Fcγ und einer Markierung verwendet. Bevorzugt ist hier ein Konjugat aus humanes Fcγ bindenden Fab Fragmenten und einer Markierung, so wie es nach der Methode von Ishikawa et.al., J. Immunoassay 4 (1983), Seite 209 erhältlich ist.

Als Fcγ bindende Fab-Fragmente können sowohl monovalente Fab- oder F(ab')-Fragmente, als auch divalente F(ab')₂-Fragmente verwendet werden.

Als Markierung können die dem Fachmann geläufigen Markierungen verwendet werden, üblicherweise wird ein Enzym, ein Fluoreszenz- oder Chemilumineszenzfarbstoff oder ein radioaktives Isotop verwendet.

Das Konjugat aus Antikörpern gegen humanes Fcγ und einer Markierung wird zunächst mit dem Kulturüberstand der immortalisierten Zellen inkubiert, danach mit humanem Immunglobulin behandelt und schließlich mit immobilisierten humanen Pankreas-Inselzellen oder immobilisierter GAD inkubiert. Als immobilisierte Inselzellen werden dabei bevorzugt Pankreasgewebeschnitte verwendet.

Die einen Antikörper gegen Pankreas-Inselzellen produzierende immortalisierte Zellinie wird dann über die Bestimmung der an die immobilisierten Inselzellen oder die immobilisierte GAD gebundenen Markierung identifiziert. Je nach Art der Markierung erfolgt diese Bestimmung über eine entsprechende Enzymreaktion oder über die Messung der Fluoreszenz oder Chemilumineszenz.

Zur Klonierung werden die Zellen, deren Kulturüberstand eine positive Reaktion zeigt, vereinzelt. Dies erfolgt vorzugsweise über einen Fluoreszenz-aktivierten Zellsorter. Die so isolierten Zellklone werden vermehrt und diejenigen Zellklone, die Antikörper gegen Pankreas-Inselzellen produzieren, wie oben beschrieben identifiziert. Aus dem Kulturüberstand dieser Zellklone wird dann der Antikörper nach dem Fachmann geläufigen Verfahren isoliert.

Bevorzugt sind solche monoklonalen Antikörper, die der Subklasse IgG1 oder IgG3 angehören.

Ein bevorzugter Gegenstand der Erfindung sind monoklonale Antikörper, die in äquivalenter Weise wie die von den Zellinien ECACC 90121401, ECACC 90121402, ECACC 90121403 oder DSM ACC 2017 produzierten Antikörper an humane Pankreas-Inselzellen oder an Glutamat-Decarboxylase bindefähig sind.

Unter dem Begriff "in äquivalenter Weise bindefähiger Antikörper" werden Antikörper verstanden, bei denen eine Epitopüberlappung mit dem definierten, bekannten Antikörper nachweisbar ist. Diese Epitopüberlappung kann mit Hilfe eines kompetitiven Testsystems leicht nachgewiesen werden. Dazu wird zum Beispiel mit Hilfe eines Enzym-Immunoassays überprüft, inwieweit ein Antikörper mit dem bekannten Antikörper um die Bindung an ein definiertes Antigen bzw. ein spezielles Epitop konkurriert. Dazu inkubiert man immobilisierte Inselzellen, z.B. Pankreasschnitte mit dem bekannten monoklonalen Antikörper in markierter Form und einem Überschuß des in Betracht gezogenen Antikörpers. Durch Nachweis der gebundenen Markierung kann dann leicht festgestellt werden, inwieweit der in Betracht gezogene Antikörper den definierten Antikörper aus der Bindung verdrängen kann. Ist eine Verdrängung von mindestens 50 % bei 10⁵-fachem Überschuß gegeben, so liegt eine Epitopüberlappung vor.

Besonders bevorzugt sind die aus den Zellinien ECACC 90121401, ECACC 90121402, ECACC 90121403 oder DSM ACC 2017 erhältlichen Antikörper.

Ein weiterer Gegenstand der Erfindung sind die Zellinien ECACC 90121401, ECACC 90121402, ECACC 90121403 und DSM ACC 2017.

Ein weiterer Gegenstand der Erfindung ist ein Verfahren zur Herstellung von humanen monoklonalen Antikörpern vom IgG-Isotyp gegen humane Pankreas Inselzellen durch Immortalisieren von humanen Lymphozyten von Prädiabetikern oder Diabetikern, Behandeln des Kulturüberstandes der immortalisierten Zellen mit einem Konjugat aus Antikörpern gegen humanes Fcγ und einer Markierung, anschließende Behandlung mit humanem Immunglobulin, Inkubieren mit immobilisierten humanen Pankreas-Inselzellen oder immobilisierter GAD, Identifizienen einer immortalisierten humanen Zellkultur, die einen Antikörper gegen Pankreas-Inselzellen produziert, über die Bestimmung der an die immobilisierten Inselzellen oder die immobilisierte GAD gebundenen Markierung, Isolieren einer humanen immortalisierten Zelle, die diesen Antikörper produziert, Vermehrung dieser immortalisierten Zelle und Isolierung des von diesen Zellen produzierten monoklonalen Antikörpers.

Mit diesem Verfahren konnten in Verbindung mit dem oben beschriebenen kompetitiven Testsystem weitere monoklonale Antikörper (HaE 12 und HAG+E 10) gegen das Inselzellantigen GAD erhalten werden, die weder mit den von den Zellinien ECACC 90121401, ECACC 90121402 und ECACC 90121403 produzierten Antikörpern noch gegenseitig eine Epitopüberlappung zeigen und somit weitere Epitope des Inselzellantigens GAD charakterisieren. Somit können aufgrund der Bestimmung der Epitopüberlappung mindestens vier Epitope des GAD-Inselzellantigens unterschieden werden, die durch die Reaktivität mit den folgenden monoklonalen Antikörpern charakterisiert sind: 1) ECACC 90121403 und ECACC 90121401, 2) ECACC 90121402, 3) HaE 12 sowie 4) HAG+E 10.

Ein weiterer Gegenstand der Erfindung ist daher ein Verfahren zur Herstellung von monoklonalen Antikörpern gegen Epitope eines Inselzellantigens, die nicht von bekannten monoklonalen Antikörpern gegen ein Inselzellantigen erkannt werden, bei dem man zunächst monoklonale Antikörper gegen ein Inselzellantigen gemäß dem erfindungsgemäßen Verfahren herstellt, diese in Gegenwart von markierten bekannten monoklonalen Antikörpern gegen ein Inselzellantigen mit immobilisierten Inselzellen inkubiert und nach dem Nachweis der gebundenen Markierung diejenigen Antikörper auswählt, die den bekannten Antikörper aus der Bindung verdrängen können.

Mit diesem Verfahren konnten die monoklonalen Antikörper HaE 12 und HaG+E 10 gewonnen werden.

Ein weiterer Gegenstand der Erfindung ist ein Verfahren zur Gewinnung des an die von den Zellinien ECACC 90121401, ECACC 90121402, ECACC 90121403 und/oder DSM ACC 2017 produzierten Antikörper bindenden Inselzellantigens durch Lysieren von humanen oder tierischen Pankreas-Inselzellen, Pankreashomogenaten, oder Gehirnhomogenaten, insbesondere Homogenaten des Kleinhirns, Abtrennung von unlöslichem Material, Behandlung der Lösung mit einem immobilisierten, aus den Zellinien ECACC 90121401, ECACC 90121402, ECACC 90121403 und/oder DSM ACC 2017 erhältlichen monoklonalen Antikörper oder einem in äquivalenter Weise an humane Pankreas Inselzellen bindenden Antikörper und Gewinnung des an die Antikörper bindenden Inselzellantigens.

Die tierischen Pankreas-Inselzellen, Pankreashomogenate oder Gehirnhomogenate können z. B. aus den entsprechenden Organen vom Schwein, Rind, der Ratte oder einer Maus gewonnen werden.

Die Immobilisierung der Antikörper erfolgt vorzugsweise durch Bindung an ein Gelmaterial wie z.B. Protein A Sepharose® CL-4B. Auf dieses Immunadsorbens wird die nach Zentrifugation erhaltene, lösliche Fraktion der genannten Gewebe nach der Lyse aufgetragen und nicht bindendes Material ausgewaschen. Anschließend wird das bindende Antigen vom Immunadsorbens eluiert. Die Elution erfolgt vorzugsweise mittels 0,05 mol/l Diethylamin pH 11,5, 0,5 % Natriumdesoxycholat.

Ein weiterer Gegenstand der Erfindung sind anti-idiotypische Antikörper, die gegen Antikörper, die mit einem Inselzellantigen reagieren, gerichtet sind, erhältlich durch Immunisierung mit einem erfindungsgemäßen Antikörper gegen ein Inselzellantigen und Isolierung der gewünschten Antikörper aus dem Serum der immunisierten Tiere durch Affinitätschromatographie. Die Immunisierung wird in den hierfür üblicherweise verwendeten Tieren, wie z.B. Schafen oder Kaninchen durchgeführt.

Ein weiterer bevorzugter Gegenstand der Erfindung sind monoklonale anti-idiotypische Antikörper, erhältlich durch Immunisierung mit einem erfindungsgemäßen Antikörper gegen ein Inselzellantigen, Immortalisierung der Milzzellen der immunisierten Tiere, Klonierung von solchen immortalisierten Zellen, die Antikörper produzieren, die an Antikörper gegen Inselzellen des Pankreas binden und Isolierung der von diesen Klonen produzierten Antikörper nach bekannten Verfahren.

Zum Nachweis von immortalisierten Zellen, die den gewünschten anti-idiotypischen Antikörper produzieren, wird der Kulturüberstand der immortalisierten Zellen mit einem immobilisierten Antikörper gegen ein Inselzellantigen inkubiert. Zum Nachweis von gebundenem anti-idiotypischen Antikörper aus dem Kulturüberstand, wird ein Konjugat aus einer Markierung und Antikörpern gegen Fcγ derjenigen Tierspezies verwendet, aus der die immortalisierten Zellen erhalten wurden. Bevorzugt ist hier ein Konjugat aus Fcγ-bindenden Fab-Fragmenten und einer Markierung, so wie es nach der Methode von Ishikawa et.al. J. Immunoassay 4 (1983), 209 erhältlich ist.

Als Fcγ bindende Fab-Fragmente können sowohl monovalente Fab- oder F(ab')-Fragmente, als auch divalente F(ab')₂-Fragmente verwendet werden.

Als Markierung können die dem Fachmann geläufigen Markierungen verwendet werden, üblicherweise wird ein Enzym, ein Floureszenz oder Chemilumineszenzfarbstoff oder ein radioaktives Isotop verwendet.

Ein weiterer Gegenstand der Erfindung ist ein Verfahren zur Herstellung von anti-idiotypischen Antikörpern durch Immunisierung mit einem erfindungsgemäßen Antikörper gegen ein Inselzellantigen und Isolierung des anti-idiotypischen Antikörpers aus dem Serum der immunisierten Tiere durch Affinitätschromatographie.

Ein weiterer bevorzugter Gegenstand der Erfindung ist ein Verfahren zur Herstellung von monoklonalen anti-idiotypischen Antikörpern durch Immunisierung mit einem erfindungsgemäßen Antikörper gegen ein Inselzellantigen, Immortalisierung der Milzzellen der immunisierten Tiere, Klonierung von solchen immortalisierten Zellen, die Antikörper produzieren, die an Antikörper gegen Inselzellen des Pankreas binden und Isolierung der von diesen Klonen produzierten Antikörper nach bekannten Verfahren.

Ein weiterer Gegenstand der Erfindung ist schließlich ein Verfahren zur Bestimmung von Antikörpern gegen ein Inselzellantigen durch Inkubation des zu untersuchenden Analysenmaterials mit einem vor oder während der Nachweisreaktion immobilisierten ersten Rezeptor und Nachweis des gebundenen, im Analysenmaterial enthaltenen Antikörpers über einen markierten, zweiten Rezeptor, der an diesen Antikörper bindet.

Als Analysenmaterial kann der Kulturüberstand von Antikörper produzierenden Zellen, ein isolierter Antikörper oder eine Körperflüssigkeit, vorzugsweise Probandenserum eingesetzt werden.

Der für den Nachweis der Antikörper im Analysenmaterial verwendete erste Rezeptor kann in Teströhrchen oder auf Mikrotiterplatten immobilisiert werden. Als erster Rezeptor kann dabei sowohl ein vollständiges Inselzellantigen, vorzugsweise das vollständige natürliche GAD-Antigen, als auch das für die Bindung verantwortliche Epitop als Fragment dieses Antigens verwendet werden.

In einer bevorzugten Ausführungsform des erfindungsgemäßen Verfahrens zur Bestimmung von Antikörpern gegen Inselzellen des Pankreas wird als immobilisierter erster Rezeptor ein anti-idiotypischer Antikörper, der das internal Image des entsprechenden Epitops trägt, verwendet.

Zur Immobilisierung wird der erste Rezeptor vorzugsweise biotinyliert und anschließend an eine mit Streptavidin beschichtete feste Phase gebunden.

In einer weiteren Ausführungsform kann der erste Rezeptor auch in Form eines Gewebeschnitts, vorzugsweise von Pankreas-Gewebe, Pankreas-Inseln oder Pankreas Inselzellen verwendet werden.

Als markierter zweiter Rezeptor wird das markierte Inselzellantigen GAD oder vorzugsweise ein markierter Antikörper gegen humanes Immunglobulin, vorzugsweise gegen humanes Fcγ verwendet. Als Markierung können alle dem Fachmann geläufigen Markierungsmethoden verwendet werden. Der Nachweis des gebundenen Probenantikörpers erfolgt nach Trennung von flüssiger und fester Phase durch Bestimmung der Markierung in der isolierten flüssigen oder festen Phase. Die Quantifizierung des Antikörpers im Analysenmaterial erfolgt durch Vergleich des Meßwerts mit dem für einen Standard-Antikörper bekannter Konzentration enthaltenen Wert.

Vorzugsweise wird als Standard-Antikörper ein erfindungsgemäßer, humaner monoklonaler Antikörper gegen humane Pankreas-Inselzellen verwendet. Hierdurch können Störungen der Bestimmung, die z.B. bei Verwendung muriner Antikörper durch Antikörper gegen Maus-Immunglobuline im Serum des Patienten hervorgerufen werden, vermieden werden.

Ein weiterer Gegenstand der Erfindung ist daher die Verwendung der erfindungsgemäßen humanen monoklonalen Antikörper als Standard für die Bestimmung von Antikörpern gegen ein Inselzellantigen im Analysenmaterial.

Ein weiterer Gegenstand der Erfindung ist schließlich ein Verfahren zur Bestimmung von Antikörpern gegen ein Inselzellantigen durch Inkubation des zu untersuchenden Analysenmaterials mit einem vor oder während der Nachweisreaktion immobilisierten ersten Rezeptor und Nachweis des gebundenen, im Analysenmaterial enthaltenen Antikörpers über einen markierten, zweiten Rezeptor, der an diesen Antikörper bindet, wobei die Menge des im Analysenmaterial enthaltenen Antikörpers bestimmt wird durch Vergleich mit dem Meßwert, der für einen erfindungsgemäßen humanen monoklonalen Antikörper erhalten wird.

Die erfindungsgemäßen Zellinien UH33/139.464 (ECACC 90121401), IID9-402 (ECACC 90121402) und AH25/43-116 (ECACC 90121403) wurden am 14.12.1990 bei der ECACC, Public Health Laboratory Service, Porton Down, Salisbury,Wiltshire SP 5 OJG, United Kingdom, hinterlegt.

Die Zellinie MAK <GAD>H-HaE 12 wurde am 21.08.1991 bei der Deutschen Sammlung von Zellkulturen und Mikroorganismen GmbH, Mascheroderweg 1 b, D-3300 Braunschweig, unter der Nummer DSM ACC 2017 hinterlegt.

Die Erfindung wird durch folgende Beispiele erläutert:

### Beispiel 1:

### Auswahl der Spender für die Lymphozytengewinnung

Für eine Auswahl der Spender, die mit hoher Wahrscheinlichkeit in vivo präaktivierte Lymphozyten gegen Inselzellantigene aufweisen, wurde folgendes Verfahren angewandt:

Prädiabetikern bzw. frisch diagnostizierten Diabetikern wurde Blut entnommen und nach bekanntem Verfahren Serum gewonnen. Dieses Serum wurde in einer Reihenverdünnung auf humanen Pankreasschnitten nach folgender Methode getestet:

25 µl des in einer geometrischen Reihe verdünnten Probandenserums wurden auf unfixierte humane Pankreasschnitte aufgebracht und zwei Stunden in einer feuchten Kammer bei Raumtemperatur inkubiert. Nach 3-maligem Waschen der Schnitte, für jeweils 5 Minuten, in PBS (phosphatgepufferte Salzlösung, nach Dulbecco und Vogt, J.Exp.Med. 99 (1954), 167-182, ohne Ca²⁺ und Mg²⁺ ) wurden 25 µl eines mit Fluorescein-Isothiocyanat markierten Antihuman-IgG-Antikörpers (5µg/ml) zugegeben und eine Stunde bei Raumtemperatur inkubiert. Nach erneutem Waschen der Schnitte wurde mit Mowiol® (Hoechst) eingebettet und die Inselzellfärbungen mit Hilfe eines Fluoreszenzmikroskops ausgewertet (Anregung bei 476 nm, Detektion bei 530 nm).

Nur solche Lymphozytenspender, deren Seren bei einer Verdünnung von mindestens oder gleich 1:64 noch eine positive Reaktion ergeben, wurden für die Isolierung von Lymphozyten verwendet.

Von den entsprechenden Spendern wurden 20 - 50 ml Blut abgenommen und daraus die mononukleären Zellen über einen Dichtegradienten isoliert. Anschließend wurden diese Zellen durch Epstein-Barr-Virus (EBV)-Transformation immortalisiert.

Dazu wurden die mononukleären Zellen in RPMI 1640 Medium (mit 10 % FKS, foetales Kälberserum) in einer Zelldichte von 2x10⁶ Zellen/ml mit 1ml des EBV enthaltenden Kulturüberstandes der B 95-8 Marmoset Zellinie (ATCC CRL 1612) für 2 Stunden bei 37°C und 5% CO₂ inkubiert. Anschließend wurden die Zellen gewaschen und 2x10⁴ Zellen je Vertiefung einer 96-er Mikrotiterplatte eingesät. Zu Beginn der Kultivierung wurde dem Medium 0,1 % Phytohaemagglutinin (Gibco, USA) zugesetzt.

### Beispiel 2:

### Klonierung der EBV-Linien

Nach drei bis vier Wochen wurden die Kulturüberstände der EBV-Linien auf Reaktivität mit Inselzellen des Pankreas getestet.

Hierzu wurden 25 µl des Kulturüberstandes zunächst mit einem Konjugat aus humanes Fcγ bindendenen Fab-Fragmenten und Peroxidase versetzt (25µl, 20 U/ml). Dieses Konjugat wurde nach der Methode von Ishikawa et al., J. Immunoassay 4 (1983) 209, präpariert. Nach einer Inkubation bei 37°C für eine Stunde wurde die Hälfte des Volumens von 10 %igem normalen Humanserum zugegeben und nochmals bei 37°C für eine Stunde inkubiert. Diese Mischung wurde auf die Pankreasschnitte aufgetragen und zwei Stunden bei 4°C inkubiert. Nach einem Waschschritt (3x5 Minuten in PBS) und 3 minütiger Vorinkubation der Schnitte in 0,1 mol/l Acetatpuffer pH 4.5 erfolgte die immunhistochemische Anfärbung durch Inkubation mit 0,02% Aminoethylcarbazol und 0,01 % H₂O₂ für 15-25 Minuten bei Raumtemperatur. Die Auswertung erfolgte unter dem Lichtmikroskop. Diejenigen EBV-Linien, deren Kulturüberstand eine positive Reaktion (Anfärbung der Inselzellen) ergab, wurden kloniert. Die Zellen wurden dazu mit Hilfe eines Fluoreszenz-aktivierten Zellsorters einzeln in 96er Mikrotiterplatten abgelegt und mit bestrahlten peripheren Blut-Lymphozyten (5 x 10⁴ Zellen/Vertiefung, 4000 rad) gefüttert. Die so erhaltenen Lymphozyten Klone werden in Iscove's modified Dulbeccos's Vollmedium (Gibco, mit 15% FKS) kultiviert. Die Antikörper werden aus dem Überstand durch Ammoniumsulfatfällung und Affinitätschromatographie, z. B. über Protein A- oder Protein-G Sepharose®, isoliert.

### Beispiel 3:

Untersuchung der Kreuzreaktivität mit verschiedenen Humangeweben und tierischen Pankreata.

Es wurde untersucht, ob die aus den Zellinien gewonnenen monoklonalen Antikörper spezifisch mit humanen Pankreas- Inselzellen reagieren. Dazu wurden die Antikörper aus dem Kulturüberstand zunächst wie in Beispiel 2 beschrieben, mit einem Konjugat aus humanes Fcγ bindenden Fab-Fragmenten und Peroxidase inkubiert. Die markierten Antikörper wurden auf einer Serie von verschiedenen humanen Gewebeschnitten sowie auf verschiedenen tierischen Pankreasschnitten getestet. Die Bindung an diese Gewebe wurde, wie in Beispiel 2 beschrieben, über immunhistochemische Anfärbung mit Aminoethylcarbazol nachgewiesen. Die Tabellen I und II zeigen die Ergebnisse dieser Tests.

**Tabelle I:**

| humane monoklonale Antikörper | | | | | | |
|---|---|---|---|---|---|---|
| humanes Gewebe | UH33 | IID9 | AH25 | HaC11 | HaE12 | HAG+E 10 |
| Pankreas | ++ | +++ | + | + | + | + |
| Nebenniere | - | - | - | - | - | - |
| Magen | - | - | - | - | - | - |
| Intestinum | - | - | - | - | - | n.b. |
| Hypophyse | - | - | - | - | - | - |
| Großhirn-Rinde | - | - | - | - | - | - |
| Lunge | - | - | - | - | - | n.b. |
| Leber | - | - | - | - | - | n.b. |
| Schilddrüse | - | - | - | - | - | - |
| Cerebellum | + | ++ | + | + | + | + |
| n.b. nicht bestimmt | | | | | | |

**Tabelle II:**

| monoklonaler Antikörper | Pankreas vom | | | |
|---|---|---|---|---|
| | Schwein | Rind | Ratte | NOD-Maus (non obese diabetic) |
| UH33 | + | + | + | + |
| IID9 | + | + | + | + |
| AH25 | + | + | + | + |
| HaC 11 | + | + | + | + |
| HaE 12 | + | + | + | + |
| HAG+E 10 | + | + | + | + |

### Beispiel 4

### Bestimmung der Kompetition von erfindungsgemäßen Antikörpern mit Diabetikerserum

Jeweils 20 µl Diabetikerserum, Serum von gesunden Kontrollpersonen bzw. PBS/BSA (PBS mit Rinderserum Albumin) werden 1:8 verdünnt auf Pankreasnormalschnitte aufgetragen und 30 Min. bei Raumtemperatur inkubiert. Danach wird 2 x 10 Min. mit PBS gewaschen. Zwischenzeitlich werden 25 µl der humanen monoklonalen Antikörper gegen das Inselzellantigen GAD mit einem Konjugat aus Peroxidase und Fab- Fragmenten vom Schaf, welche humane Fcγ-Fragmente binden, versetzt (25 µl; 20 U/ml). Dieses Konjugat wurde nach der Methode von Ishikawa et.al. J. Immunoassay 4 (1983), Seite 209 präpariert. Diese präformierten Immunkomplexe werden auf die mit den Seren vorinkubierten Pankreasnormalschnitte aufgetragen, 2 Std. bei 4°C inkubiert und 3 x 10 Min. mit PBS gewaschen. Die Peroxidasefärbung wird wie in Beispiel 2 angegeben mit Aminoethylcarbazol durchgeführt und anschließend im Lichtmikroskop bewertet, ob die Färbung im Vergleich zur Vorinkubation mit PBS/BSA bei Vorinkubation mit den einzelnen Seren abgeschwächt wurde oder nicht.

Dabei zeigt sich, daß Normalseren keinen Einfluß auf die anschließende Bindung der monoklonalen Antikörper gegen Inselzellen auf den Schnitt haben, während die drei Diabetikerseren in unterschiedlicher Weise die Bindung der monoklonalen Antikörper gegen Inselzell-Antigene hemmen. Dies deutet auf Antikörper in den Diabetikerseren hin, die das selbe oder ein sehr eng benachbartes Epitop erkennen, wie die erfindungsgemäßen monoklonalen Antikörper gegen Inselzellen. D.h., daß die hier isolierten monoklonalen Antikörper gegen Inselzellen in mehreren Diabetikerseren vorhandene Antikörper repräsentieren und die zugehörigen Epitope für die Diabeteserkrankung relevanten Autoantigenen zugehören.

**Tabelle III:**

| Vorinkubation mit | ICA-Spiegel JDF-Einheiten | Reaktionen von Pankreaseschnitten | | | | | |
|---|---|---|---|---|---|---|---|
| | | AH25 | HaC11 | UH33 | HaE12 | IID9 | HAG+E10 |
| PBS/BSA | - | ++ | + | + | +++ | +++ | ++ |
| Diabetikerserum 1 | 1280 | (+) | - | - | - | - | - |
| Diabetikerserum 2 | 640 | + | + | (+) | - | + | (+) |
| Diabetikerserum 3 | 160 | + | + | - | + | (+) | + |
| Kontrollserum 1 | - | ++ | + | ++ | +++ | +++ | ++ |
| Kontrollserum 2 | - | ++ | + | ++ | +++ | +++ | ++ |

### Beispiel 5

### Bestimmung der Epitopüberlappung von Antikörpern gegen Inselzellen des Pankreas

Zum Nachweis der Epitopüberlappung eines Antikörpers mit dem monoklonalen Antikörper ECACC 90121401, ECACC 90121402, ECACC 90121403 oder DSM ACC 2017 wurde ein kompetitiver Enzymimmunoassay durchgeführt.

Der zu beurteilende Antikörper wurde in verschiedenen Verdünnungsstufen 1 Std. bei Raumtemperatur mit humanen Pankreasschnitten inkubiert und 3 x 5 Min. in PBS gewaschen. Anschließend werden Peroxidase markierte Immunkomplexe aus den oben genannten monoklonalen Antikörpern mit einem Konjugat aus humanes Fcγ-bindenden Fab-Fragmenten und Peroxidase (Herstellung wie in Beispiel 2 beschrieben) auf die Pankreasschnitte gegeben und 2 Std. bei 4°C inkubiert. Nach erneutem Waschen (3 x 5 Min. in PBS) erfolgt der Nachweis von gebundenem Peroxidase markiertem Antikörper ECACC 90121401, ECACC 90121402, ECACC 90121403 bzw. DSM ACC 2017 durch immunhistochemische Anfärbung mit Aminoethylcarbazol (so wie in Beispiel 2 beschrieben). Die erhaltene Färbung wurde verglichen mit der Färbung, die erhalten wurde bei Inkubation mit dem monoklonalen Antikörper ECACC 90121401, ECACC 90121402, ECACC 90121403 oder DSM ACC 2017 allein.

Wenn bis zu einem 10⁵-fachen Überschuß an zu beurteilendem Antikörper gegenüber dem monoklonalen Antikörper ECACC 90121401, ECACC 90121402, ECACC 90121403 oder DSM ACC 2017 mindestens 50 % Kompetition zu erkennen sind, liegt eine Epitopüberlappung vor.

Auf diese Weise konnte gezeigt werden, daß ein weiterer Antikörper gegen ein Inselantigen des Pankreas, HaC 11, ein Epitop erkennt, daß mit dem vom Antikörper ECACC 90121402 erkannten Epitop überlappt.

### Beispiel 6

### Isolierung von Inselzellantigen durch Immunaffinitäts-chromatographie

### a) Herstellung des Immunadsorbens

Protein A - Sepharose® CL-4B wird in 0,1 mol/l Boratpuffer pH 8,2 mit dem humanen monoklonalen Antikörper (ECACC 90121401, ECACC 90121402, ECACC 90121403 oder DSM ACC 2017; 1 ml Sepharose bindet 20 mg Antikörper) bei Raumtemperatur für eine Stunde vorsichtig geschüttelt. Die Sepharose® wird dann mit 0,1 mol/l Boratpuffer pH 8,2 proteinfrei gewaschen und in eine Säule gefüllt. Das Säulenmaterial wird mit 0,15 mol/l NaCl; 0,01 mol/l Tris-HCl pH 8,2; 1 mmol/l EDTA; 0,5 % Triton® X-100 äquilibriert.

### b) Isolierung des Inselzellantigens

Durch limitierte Proteolyse von Pankreasgewebe und Dichtezentrifugation erhaltene, isolierte humane Pankreas-Inselzellen werden durch Behandlung mit 0,15 mol/l NaCl; 0,01 mol/l Tris HCl pH 8,2; 1 mmol/l EDTA; 0,5 % Triton® X-100; 2 mmol/l Phenylmethylsulfonylfluorid für eine halbe Stunde auf Eis lysiert. Nach Abtrennung von Zellpartikeln durch Zentrifugation bei 20000 g wird das Lysat langsam (0,2 ml/min) über die nach a) hergestellte Immunadsorbens-Säule gepumpt. Die Säule wird anschließend nacheinander mit folgenden Lösungen gewaschen (jeweils 10 Säulenvolumen):
a): 0,5 mol/l NaCl; 0,05 mol/l Tris HCl, pH 8,2; 1 mmol/l EDTA; 0,5 % Triton® X-100
b):0,15 mol/l NaCl; 0,5 % Natriumdesoxycholat
c):0,15 mol/l NaCl; 0,01 mol/l Tris HCl pH 8,2; 1 mmol/l EDTA; 0,05 % Triton® X-100.

Das an das Immunadsorbens gebundene Antigen wird mittels 0,05 mol/l Diethylamin pH 11,5, 0,5 % Natrimdesoxycholat eluiert. Ein Aliquot der Säuleneluate wird adsorptiv an eine Festphase (Nitrocellulose oder Mikrotiterplatte) gebunden und mit der Lösung eines isolierten von den Zellinien ECACC 90121401, ECACC 90121402, ECACC 90121403 oder DSM ACC 2017 der produzierten Inselzell-Antikörpers (1 µg/ml) für 2 Stunden bei Raumtemperatur inkubiert und anschließend mit 3 x 350 µl 0,15 mol/l NaCl/0,05 % Tween® 20 gewaschen. Zum Nachweis von gebundenem Antikörper wird mit 100 µl eines Peroxidase -markierten Schaf-Antihuman-Fcγ - Antikörpers (Boehringer Mannheim, Kat. Nr. 1 089 196, 100 mU/ml in PBS/0,5% BSA) für 1 Stunde bei Raumtemperatur unter Schütteln inkubiert und anschließend 3 x mit 350 µl 0,15 mol/l NaCl/0,05% Tween® 20 gewaschen. Danach werden 100 µl ABTS® (lmg/ml, Boehringer Mannheim GmbH, Kat.Nr. 756 407) in 40 mmol/l Citrat-Puffer pH 4,4, der 3,25 mmol/l Natriumperborat enthält, zugegeben und nach 30 minütiger Inkubation bei Raumtemperatur die Extinktion bei 405 nm gemessen.

Die positiv reagierenden Fraktionen werden vereinigt, durch Zugabe von 0,5 mol/l NaH₂PO₄ neutralisiert und danach gegen 0,15 mol/l NaCl; 0,01 mol/l Tris-HCl, pH 8,2; 1 mmol/l EDTA; 0,05 % Triton® X-100 dialysiert.

### Beispiel 7:

Bestimmung von Inselzell-Antikörpern (ICA) mit ELISA-Technik:
a) Beschichten von Mikrotiterplatten oder Teströhrchen mit dem ersten Rezeptor
   Der erste Rezeptor (z.B. Inselzellantigen GAD) wird über seine Aminogruppen mit D-Biotinyl-E-amidocapronsäure-N-hydroxysuccinimidester (Boehringer Mannheim GmbH, Kat.Nr. 1008 960) oder über Tyrosinreste mit Diazo-para-aminobenzoylbiocytin (DBB, Calbiochem) gemäß Angaben des Herstellers biotinyliert.
   Anschließend werden 150 µl des biotinylierten Rezeptors (0,5 µg/ml in PBS ohne Mg²⁺ und Ca²⁺) mit einer Streptavidin beschichteten festen Phase (Mikrotiterplatten oder Teströhrchen, Herstellung nach EP-A 0 344 578) 30 Minuten bei Raumtemperatur inkubiert und mit 0,15 mol/l NaCl/0,05 % Tween® 20 gewaschen.
b)Inkubation mit dem Analysenmaterial
   Probandenserum wird 1:25 mit PBS, das 0,5 % BSA (Rinder-Serumalbumin) enthält, verdünnt und 100 pl dieser Verdünnung zu dem nach a) immobilisierten Rezeptor pipettiert. Als Positivkontrolle werden 100 µl eines isolierten Inselzellantikörpers (1 µg/ml PBS/0,5% BSA) eingesetzt. Anschließend wird 2 Stunden bei Raumtemperatur unter Schütteln inkubiert und schließlich mit 3 x 350 µl 0,15 mol/l NaCl/0,05 % Tween® 20 gewaschen.
c)Nachweisreaktion
   Zum Nachweis von gebundenen ICA aus dem Analysenmaterial wird mit 100 µl eines Peroxidase - markierten Schaf-Antihuman-Fcγ - Antikörpers (Boehringer Mannheim GmbH, Kat. Nr. 1 089 196, 100 mU/ml in PBS/0,5% BSA) für 1 Stunde bei Raumtemperatur unter Schütteln inkubiert und anschließend 3 x mit 350 µl 0,15 mol/l NaCl/0,05% Tween® 20 gewaschen. Danach werden 100 µl ABTS® (1mg/ml, Boehringer Mannheim GmbH, Kat.Nr. 756 407) in 40 mmol/l Citrat-Puffer pH 4,4, der 3,25 mmol/l Natriumperborat enthält, zugegeben und nach 30 minütiger Inkubation bei Raumtemperatur die Extinktion bei 405 nm gemessen.

### Beispiel 8:

### Bestimmung der Reaktion von humanen monoklonalen Antikörpern mit gereinigter Glutamat-Decarboxylase (GAD) aus Schweinehirn

Die GAD wurde aus Schweinehirn nach der Methode von Spink et al. (J. Neurochemistry, 40, 4, (1983) 1113), gereinigt. Für die nachstehend beschriebenen Versuche wurde eine Fraktion aus der Sepharose S-200 Gelfiltration verwendet, in welcher das Enzym in ca. 90 %iger Reinheit vorlag.

Das Enzym wurde in Beschichtungspuffer (Boehringer Mannheim GmbH, Katalog-Nr. 726559) auf eine Konzentration von 10 µg/ml gebracht und in Nunc Maxisorp® Immunoplatten pipettiert (50 µl/Vertiefung). Nach einer Stunde Inkubation bei 37°C wurde die Beschichtungslösung abpipettiert und mit 1 % Crotein C in PBS unspezifische Bindungsstellen der Mikrotiterplatte abgesättigt (200 µl/Vertiefung; 1 Stunde bei 37°C).

Zwischenzeitlich wurden 25 µl der Kulturüberstände, welche die entsprechenden humanen monoklonalen Antikörper enthielten, mit einem Peroxidase-Konjugat aus Fab-Fragmenten vom Schaf, welche humane Fcγ-Fragmente binden, versetzt (25 µl; 20 U/ml). Dieses Konjugat wurde nach der Methode von Ishikawa et al., J. Immunoassay 4 (1983) 209, präpariert. Nach einer Inkubation bei 37°C für eine Stunde wurde die Hälfte des Volumens von 10 %igem normalen Humanserum zugegeben und nochmals bei 37°C für eine Stunde inkubiert.

Diese Mischung wurde in die Antigen-beschichteten und gewaschenen Vertiefungen der Mikrotiterplatte gegeben und zwei Stunden bei 4°C inkubiert.

Nach einem Waschschritt (3x mit PBS/0,05 % Tween® 20) wurden 100 µl ABTS® (1 mg/ml in 40 mmol/l Citratpuffer, pH 4,4, der 3,25 mmol/l Natriumperborat enthält), zugegeben und nach 45 minütiger Inkubation bei Raumtemperatur die Extinktion bei 405 nm gemessen.

Tabelle III zeigt die Ergebnisse der Reaktion der verwendeten monoklonalen Antikörper mit gereinigter Schweinehirn-GAD.

| monoklonaler Antikörper | mE 405 nm | Antikörperkonzentration (µg/ml) |
|---|---|---|
| AH 25 (ECACC 90121403) | 694 | 2,0 |
| UH 33 (ECACC 90121401) | 458 | 3,3 |
| II D9 (ECACC 90121402) | 691 | 22,0 |
| H-IgG 1 Myelomprotein (Negativkontrolle) | 10 | 20,0 |

## Patentansprüche

1. Humane monoklonale Antikörper gegen humane Pankreas-Inselzellen, dadurch gekennzeichnet, daß sie vom IgG-Isotyp sind und mit gereinigter GAD reagieren.

2. Humane monoklonale Antikörper nach Anspruch 1, erhältlich durch Immortalisieren von humanen Lymphozyten von Prädiabetikern oder Diabetikern, Behandeln des Kulturüberstandes der immortalisierten Zellen mit einem Konjugat aus Antikörpern gegen humanes Fcy und einer Markierung, anschließende Behandlung mit humanem Immunglobulin, Inkubieren mit immobilisierten humanen Pankreas-Inselzellen oder immobilisierter GAD, Identifizieren einer immortalisierten humanen Zellkultur, die einen Antikörper gegen Pankreas-Inselzellen produziert, über die Bestimmung der an die immobilisierten Inselzellen oder die immobilisierte GAD gebundenen Markierung, Isolieren einer humanen immortalisierten Zelle, die diesen Antikörper produziert, Vermehrung dieser immortalisierten Zelle und Isolierung des von diesen Zellen produzierten monoklonalen Antikörpers.

3. Humane monoklonale Antikörper gemäß Ansprüchen 1 und 2, dadurch gekennzeichnet, daß sie den Subklassen IgG1 oder IgG3 angehören.

4. Humane monoklonale Antikörper gemäß Ansprüchen 1 bis 3, die in äquivalenter Weise bindefähig sind, wie die Antikörper, die von den Zellinien ECACC 90121401, ECACC 90121402, ECACC 90121403 oder DSM ACC 2017 produziert werden, wobei unter in äquivalenter Weise bindefähig verstanden wird, daß eine Epitopüberlappung mit den bekannten, definierten Antikörpern existiert, wobei die Epitopüberlappung dann vorliegt, wenn in einem kompetitiven Testsystem die bekannten, definierten Antikörper bei einem 10⁵-fachen Überschuß die monoklonalen Antikörper mindestens zu 50 % aus ihrer Bindung an ein definiertes Antigen bzw. ein spezielles Epitop verdrängen.

5. Humane monoklonale Antikörper gemäß Ansprüchen 1 bis 4, erhältlich aus den Zellinien ECACC 90121401, ECACC 90121402, ECACC 90121403 oder DSM ACC 2017.

6. Zellinie ECACC 90121401, ECACC 90121402, ECACC 90121403 oder DSM ACC 2017.

7. Verfahren zur Herstellung von humanen monoklonalen Antikörpern nach einem der Ansprüche 1-5 durch Immortalisieren von humanen Lymphozyten von Prädiabetikern oder Diabetikern, behandeln des Kulturüberstandes der immortalisierten Zellen mit einem Konjugat aus Antikörpern gegen humanes Fcy und einer Markierung, anschließende Behandlung mit humanem Immunglobulin, Inkubieren mit immobilisierten humanen Pankreas-Inselzellen oder immobilisierte GAD, Identifizieren einer immortalisierten humanen Zellkultur, die einen Antikörper gegen Pankreas-Inselzellen oder die immobilisierte GAD produziert, über die Bestimmung der an die immobilisierten Inselzellen gebundenen Markierung, Isolieren einer humanen immortalisierten Zelle, die diesen Antikörper produziert, Vermehrung dieser immortalisierten Zelle und Isolierung des von diesen Zellen produzierten monoklonalen Antikörpers.

8. Verfahren zur Herstellung monoklonaler Antikörper gegen Epitope eines Inselzellantigens, die nicht von bekannten monoklonalen Antikörpern gegen ein Inselzellantigen erkannt werden, bei dem man zunächst monoklonale Antikörper gegen ein Inselzellantigen nach dem Verfahren gemäß Anspruch 7 herstellt, diese in Gegenwart von markierten bekannten monoklonalen Antikörpern gegen ein Inselzellantigen zusammen mit Inselzellen inkubiert und nach dem Nachweis der gebundenen Markierung diejenigen Antikörper auswählt, die den bekannten Antikörper aus der Bindung verdrängen können.

9. Verwendung eines monoklonalen Antikörpers gemäß Anspruch 4 zur Gewinnung von Inselzellantigenen aus lysiertem humanen oder tierischen Pankreas-Inselzellen, Pankreas-Homogenaten, Gehirn- oder Kleinhirnhomogenaten.

10. Anti-idiotypischer Antikörper, der gegen Antikörper, die mit Pankreas-Inselzellen reagieren, gerichtet ist, erhältlich durch Immunisierung mit einem Antikörper gegen eine Inselzellantigen gemäß einem der Ansprüche 1-5 und Isolierung des anti-idiotypischen Antikörpers aus dem Serum der immunisierten Tiere durch Affinitätschromatographie.

11. Monoklonaler anti-idiotypischer Antikörper, der gegen Antikörper, die mit Pankreas-Inselzellen reagieren, gerichtet ist, erhältlich durch Immunisierung mit einem Antikörper gegen ein Inselzellantigen gemäß einem der Ansprüche 1-5, Immortalisierung der Milzzellen der immunisierten Tiere, Klonierung von solchen immortalisierten Zellen, die Antikörper produzieren, die an Antikörper gegen Inselzellen des Pankreas binden und Isolierung der von diesen Klonen produzierten Antikörper nach bekannten Verfahren.

12. Verfahren zur Herstellung von anti-idiotypischen Antikörpern durch Immunisierung mit einem Antikörper gegen ein Inselzellantigen gemäß einem der Ansprüche 1-5 und Isolierung des anti-idiotypischen Antikörpers aus dem Serum der immunisierten Tiere duch Affinitätschromatographie.

13. Verfahren zur Herstellung von monoklonalen anti-idiotypischen Antikörpern durch Immunisierung mit einem Antikörper gegen ein Inselzellantigen gemäß einem der Ansprüche 1-5, Immortalisierung der Milzzellen der immunisierten Tiere, Klonierung von solchen immortalisierten Zellen, die Antikörper produzieren, die an Antikörper gegen Inselzellen des Pankreas binden und Isolierung der von diesen Klonen produzierten Antikörper nach bekannten Verfahren.

14. Verwendung eines anti-idiotypischen Antikörpers gemäß einem der Ansprüche 10 oder 11 als immobilisierter Rezeptor, in einem Verfahren zur Bestimmung von Antikörpern gegen ein Inselzellantigen.

15. Verwendung von humanen monoklonalen Antikörpern gegen Inselzellen des Pankreas gemäß einem der Ansprüche 1-5 allein oder in Kombination als Standard in einem Verfahren zur Bestimmung von Antikörpern gegen ein Inselzellantigen.

## Claims

1. Human monoclonal antibodies against human pancreatic islet cells, wherein they are of the IgG isotype and react with purified GAD.

2. Human monoclonal antibodies as claimed in claim 1 which can be obtained by immortalizing human lymphocytes of prediabetics or diabetics, treating the culture supernatant of the immortalized cells with a conjugate of antibodies against human Fcy and a label, subsequently treating with human immunoglobulin, incubating with immobilized human pancreatic islet cells or immobilized GAD, identifying an immortalized human cell culture which produces an antibody against pancreatic islet cells via determination of the label bound to the immobilized islet cells or to the immobilized GAD, isolating a human immortalized cell which produces this antibody, propagating this immortalized cell and isolating the monoclonal antibody produced by these cells.

3. Human monoclonal antibodies as claimed in claims 1 and 2, wherein they belong to the subclasses IgGl or IgG3.

4. Human monoclonal antibodies as claimed in claims 1 to 3 which are capable of binding in an equivalent manner to the antibodies which are produced by the cell lines ECACC 90121401, ECACC 90121402, ECACC 90121403 or DSM ACC 2017, wherein capable of binding in an equivalent manner is understood to mean that there is an epitope overlap with the known defined antibodies, an epitope overlap being then present when in a competitive test system, the known defined antibodies at a 10⁵-fold excess displace the monoclonal antibodies by at least 50 % from their binding to a defined antigen or a special epitope.

5. Human monoclonal antibodies as claimed in claims 1 to 4 obtainable from the cell lines ECACC 90121401, ECACC 90121402, ECACC 90121403 or DSM ACC 2017.

6. Cell line ECACC 90121401, ECACC 90121402, ECACC 90121403 or DSM ACC 2017.

7. Process for the production of human monoclonal antibodies as claimed in one of the claims 1-5 by immortalizing human lymphocytes of prediabetics or diabetics, treating the culture supernatant of the immortalized cells with a conjugate of antibodies against human Fcy and a label, subsequently treating with human immunoglobulin, incubating with immobilized human pancreatic islet cells or immobilized GAD, identifying an immortalized human cell culture which produces an antibody against pancreatic islet cells or the immobilized GAD via determination of the label bound to the immobilized islet cells, isolating a human immortalized cell which produces this antibody, propagating this immortalized cell and isolating the monoclonal antibody produced by these cells.

8. Process for the production of monoclonal antibodies against epitopes of an islet cell antigen which are not recognized by known monoclonal antibodies against an islet cell antigen in which firstly monoclonal antibodies against an islet cell antigen are produced according to the process as claimed in claim 7, these are incubated in the presence of labelled known monoclonal antibodies against an islet cell antigen together with islet cells and, after detecting the bound label, those antibodies are selected which can displace the known antibody from the binding.

9. Use of a monoclonal antibody as claimed in claim 4 to isolate islet cell antigens from lysed human or animal pancreatic islet cells, pancreatic homogenates or homogenates of brain or cerebellum.

10. Anti-idiotypic antibody which is directed against antibodies which react with pancreatic islet cells obtainable by immunizing with an antibody against an islet cell antigen as claimed in one of the claims 1 to 5 and isolating the anti-idiotypic antibody from the serum of the immunized animals by affinity chromatography.

11. Monoclonal anti-idiotypic antibody which is directed against antibodies which react with pancreatic islet cells obtainable by immunizing with an antibody against an islet cell antigen as claimed in one of the claims 1 to 5, immortalizing the spleen cells of the immunized animals, cloning those immortalized cells which produce antibodies that bind to antibodies against islet cells of the pancreas and isolating the antibodies produced by these clones according to known methods.

12. Process for the production of anti-idiotypic antibodies by immunizing with an antibody against an islet cell antigen as claimed in one of the claims 1 to 5 and isolating the anti-idiotypic antibody from the serum of the immunized animals by affinity chromatography.

13. Process for the production of monoclonal anti-idiotypic antibodies by immunizing with an antibody against an islet cell antigen as claimed in one of the claims 1 to 5, immortalizing the spleen cells of the immunized animals, cloning those immortalized cells which produce antibodies that bind to antibodies against islet cells of the pancreas and isolating the antibodies produced by these clones according to known methods.

14. Use of an anti-idiotypic antibody as claimed in one of the claims 10 or 11 as an immobilized receptor in a method for the determination of antibodies against an islet cell antigen.

15. Use of human monoclonal antibodies against islet cells of the pancreas as claimed in one of the claims 1 to 5 on their own or in combination as a standard in a method for the determination of antibodies against an islet cell antigen.

## Revendications

1. Anticorps monoclonaux humains contre des cellules d'îlots du pancréas, humaines, caractérisés en ce qu'ils sont de l'isotype IgG et réagissent avec la GAD purifiée.

2. Anticorps monoclonaux humains selon la revendication 1, pouvant être obtenus par immortalisation de lymphocytes humains de prédiabétiques ou diabétiques, traitement du surnageant de culture des cellules immortalisées avec un conjugué d'anticorps contre le Fcy humain et un marqueur, traitement subséquent avec de l'immunoglobuline humaine, incubation avec des cellules d'îlots du pancréas, humaines, immmobilisées ou la GAD immobilisée, identification d'une culture de cellules humaines immortalisées, qui produit un anticorps contre des cellules d'îlots du pancréas, par la détermination du marqueur lié aux cellules d'îlots immobilisées ou à la GAD immobilisée, isolement d'une cellule immortalisée humaine, qui produit cet anticorps, multiplication de cette cellule immortalisée et isolement de l'anticorps monoclonal produit à partir de ces cellules.

3. Anticorps monoclonaux humains selon les revendications 1 et 2, caractérisés en ce qu'ils appartiennent aux sous-classes IgG1 ou IgG3.

4. Anticorps monoclonaux humains selon les revendications 1 à 3, qui sont capables de liaison d'une manière équivalente à celle des anticorps qui sont produits à partir des lignées de cellules ECACC 90121401,ECACC 90121402, ECACC 90121403 ou DSM ACC 2017, où il est entendu par "capables de liaison d'une manière équivalente" qu'un recouvrement d'épitopes avec les anticorps définis, connus existe, où on se trouve alors en présence du recouvrement d'épitopes lorsque, dans un système d'analyse compétitive, les anticorps définis, connus, déplacent avec un excès de 10⁵ les anticorps monoclonaux d'au moins 50% de leur liaison à un antigène défini ou un épitope spécial.

5. Anticorps monoclonaux humains selon les revendications 1 à 4, pouvant être obtenus à partir des lignées de cellules ECACC 90121401, ECACC 90121402, ECACC 90121403 ou DSM ACC 2017.

6. Lignée de cellules ECACC 90121401, ECACC 90121402, ECACC 90121403 ou DSM ACC 2017.

7. Procédé de production d'anticorps monoclonaux humains selon l'une des revendications 1-5 par immortalisation de lymphocytes humains de prédiabétiques ou diabétiques, traitement du surnageant de culture des cellules immortalisées avec un conjugué d'anticorps contre le Fcy humain et un marqueur, traitement subséquent avec de l'immunoglobuline humaine, incubation avec des cellules d'îlots du pancréas humaines, immobilisées ou la GAD immobilisée, identification d'une culture de cellules humaines immortalisées, qui produit un anticorps contre des cellules d'îlots du pancréas ou la GAD immobilisée, par la détermination du marqueur lié aux cellules d'îlots immobilisées, isolement d'une cellule immortalisée humaine, qui produit cet anticorps, multiplication de cette cellule immortalisée et isolement de l'anticorps monoclonal produit à partir de ces cellules.

8. Procédé de production d'anticorps monoclonaux contre des épitopes d'un antigène de cellule d'îlot, qui ne sont pas reconnus par des anticorps monoclonaux connus contre un antigène de cellule d'îlot, dans lequel on produit tout d'abord des anticorps monoclonaux contre un antigène de cellule d'îlot selon le procédé conforme à la revendication 7, met ceux-ci à incuber en présence d'anticorps monoclonaux, connus, marqués, dirigés contre un antigène de cellule d'îlot, conjointement avec des cellules d'îlots, et on sélectionne après dosage du marqueur lié les anticorps qui peuvent déplacer de la liaison l'anticorps connu.

9. Utilisation d'un anticorps monoclonal selon la revendication 4 pour la production d'antigènes de cellules d'îlots à partir d'un homogénat du cervelet ou du cerveau, homogénat du pancréas, de cellules d'îlots du pancréas animaux ou humains, lysé.

10. Anticorps anti-idiotypique, qui est dirigé contre des anticorps qui réagissent avec des cellules d'îlots du pancréas, pouvant être obtenu par immunisation avec un anticorps contre un antigène de cellule d'îlot selon l'une des revendications 1-5 et isolement de l'anticorps anti-idiotypique à partir du sérum des animaux immunisés, par chromatographie d'affinité.

11. Anticorps anti-idiotypique monoclonal qui est dirigé contre des anticorps qui réagissent avec des cellules d'îlots du pancréas, pouvant être obtenu par immunisation avec un anticorps contre un antigène de cellule d'îlot selon l'une des revendications 1-5, immortalisation des cellules spléniques des animaux immunisés, clonage de telles cellules immortalisées qui produisent des anticorps, qui se lient à des anticorps contre des cellules d'îlots du pancréas et isolement des anticorps produits à partir de ces clones, selon des procédés connus.

12. Procédé de production d'anticorps anti-idiotypiques par immunisation avec un anticorps contre un antigène de cellule d'îlot selon l'une des revendications 1-5 et isolement de l'anticorps anti-idiotypique à partir du sérum des animaux immunisés, par chromatographie d'affinité.

13. Procédé de production d'anticorps anti-idiotypiques monoclonaux par immunisation avec un anticorps contre un antigène de cellule d'îlot selon l'une des revendications 1-5, immortalisation des cellules spléniques des animaux immunisés, clonage de telles cellules immortalisées qui produisent des anticorps, qui se lient à des anticorps contre des cellules d'îlots du pancréas et isolement des anticorps produits à partir de ces clones, selon des procédés connus.

14. Utilisation d'un anticorps anti-idiotypique selon l'une des revendications 10 ou 11 comme récepteur immobilisé, dans un procédé de détermination d'anticorps contre un antigène de cellule d'îlot.

15. Utilisation d'anticorps monoclonaux humains contre des cellules d'îlots du pancréas selon l'une des revendications 1-5, seule ou en combinaison comme étalon dans un procédé de détermination d'anticorps contre un antigène de cellule d'îlot.
